(19) Europäisches Patentamt
European Patent Office
Office européen des brevets

(11) **EP 3 752 864 B1**

(12) **EUROPEAN PATENT SPECIFICATION**

(45) Date of publication and mention
of the grant of the patent:
**05.07.2023 Bulletin 2023/27**

(21) Application number: **19840996.3**

(22) Date of filing: **23.07.2019**

(51) International Patent Classification (IPC):
**G01P 15/08** (2006.01)   **G01T 1/161** (2006.01)
**G01L 5/00** (2006.01)   **G01T 7/00** (2006.01)
**A61B 6/10** (2006.01)

(52) Cooperative Patent Classification (CPC):
**G01P 15/0891; A61B 6/102; A61B 6/4283;**
A61B 6/4405

(86) International application number:
**PCT/KR2019/009078**

(87) International publication number:
**WO 2020/022743 (30.01.2020 Gazette 2020/05)**

(54) **X-RAY DETECTOR INCLUDING SHOCK-DETECTING SENSOR, X-RAY SYSTEM INCLUDING X-RAY DETECTOR, AND METHOD OF OPERATING X-RAY SYSTEM**

RÖNTGENDETEKTOR MIT STOSSERKENNUNGSSENSOR, RÖNTGENSYSTEM MIT RÖNTGENDETEKTOR UND VERFAHREN ZUM BETRIEB DES RÖNTGENSYSTEMS

DÉTECTEUR DE RAYONS X COMPRENANT UN CAPTEUR DE DÉTECTION DE CHOC, SYSTÈME DE RAYONS X COMPRENANT UN DÉTECTEUR DE RAYONS X ET PROCÉDÉ DE FONCTIONNEMENT D'UN SYSTÈME DE RAYONS X

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.07.2018 KR 20180086020**

(43) Date of publication of application:
**23.12.2020 Bulletin 2020/52**

(73) Proprietor: **Samsung Electronics Co., Ltd.
Gyeonggi-do 16677 (KR)**

(72) Inventors:
• **KIM, Youngik
Suwon-si, Gyeonggi-do 16677 (KR)**
• **CHO, Minsik
Suwon-si, Gyeonggi-do 16677 (KR)**
• **KIM, Uncheol
Suwon-si, Gyeonggi-do 16677 (KR)**

(74) Representative: **Arnold & Siedsma
Bezuidenhoutseweg 57
2594 AC The Hague (NL)**

(56) References cited:
KR-A- 20130 092 007   US-A1- 2006 071 172
US-A1- 2007 138 400   US-A1- 2011 038 111
US-A1- 2012 133 600   US-A1- 2013 205 868
US-A1- 2015 359 505   US-A1- 2015 359 505
US-A1- 2018 156 680   US-B1- 8 113 045

## Description

### Technical Field

The disclosure relates to X-ray detectors, X-ray systems including X-ray detectors, and methods of operating the X-ray systems, and more particularly, to X-ray detectors which include shock-detecting sensors for detecting a shock applied to the X-ray detectors and provide information about the shock to a user, and X-ray systems including the X-ray detectors.

### Background Art

X-rays are electromagnetic waves having wavelengths of 0.01 to 100 angstroms (Å), and are widely used, due to their ability to penetrate objects, in medical apparatuses for imaging the inside of a living body or in non-destructive testing equipment for industrial use.

A basic principle of an X-ray imaging apparatus using X-rays is that an internal structure of an object is examined by transmitting X-rays emitted from an X-ray tube (or an X-ray source) through an object and detecting a difference in intensities of the transmitted X-rays via an X-ray detector. As portable X-ray detectors have recently been developed and have come into widespread use, the portable X-ray detectors are prone to being dropped or experiencing shocks during X-ray imaging. Thus, an outer casing of an X-ray detector may have a shock-absorbing member attached thereto or may be formed of a material capable of easily absorbing shock.

A shock-detecting sensor may be positioned within an X-ray detector and detect a fall of the X-ray detector or an impact externally applied to the X-ray detector and measure an impulse occurring due to the impact. However, due to recent increased compactness and simplification of portable devices, the X-ray detector has a small size, and a shock-detecting sensor mounted inside the X-ray detector also has a small size. Thus, a range of impulses measurable by the shock-detecting sensor is limited. In other words, when a shock above a certain range is applied to the X-ray detector, a measurement value from the shock-detecting sensor is saturated, and thus, an impulse applied to the X-ray detector cannot be accurately calculated.

Reference is made here to US 2018/0156680 A1, which is acknowledged here to constitute the closest prior-art and discloses a radiography image photographing device including an acceleration sensor. Moreover, reference is made here to US 2006/0071172 A1 disclosing an X-ray detector having a cover assembly capable of absorbing high-energy impacts to inhibit fracturing of internal components of the X-ray detector. In addition, US 8 113 045 B1, US 2015/359505 A1 and US 2013/205868 A1 are acknowledged here as further prior-art bearing at least some relevance to the present invention.

## Disclosure of Invention

### Solution to Problem

Provided is an X-ray system exhibiting features specified in the appended independent product claim.

Provided is also a method of operating the X-ray system in accordance with the present invention as defined in the appended independent method claim.

Additional aspects will be set forth in part in the description which follows and, in part, will be apparent from the description, or may be learned by practice of the presented embodiments of the disclosure.

### Brief Description of Drawings

The above and other aspects, features, and advantages of certain embodiments of the disclosure will be more apparent from the following description taken in conjunction with the accompanying drawings, in which:

FIG. 1 is a perspective diagram showing a configuration of an X-ray apparatus according to an embodiment of the disclosure;

FIG. 2 is an external view of an X-ray detector according to an embodiment of the disclosure;

FIG. 3 is an external view and block diagram of an X-ray apparatus implemented as a mobile X-ray apparatus, according to an embodiment of the disclosure;

FIG. 4A is an exploded perspective view showing components of an X-ray detector according to an embodiment of the disclosure;

FIG. 4B is an enlarged view of a part of the X-ray detector;

FIG. 5A is an exploded perspective view showing some components of an X-ray detector according to an embodiment of the disclosure;

FIG. 5B is a cross-sectional view illustrating some components of the X-ray detector;

FIG. 6A is a cross-sectional view illustrating some components of an X-ray detector according to an embodiment of the disclosure;

FIG. 6B is a plan view of the components illustrated in FIG. 6A;

FIG. 7 is a cross-sectional view illustrating some components of an X-ray detector according to an embodiment of the disclosure;

FIG. 8A is a cross-sectional view illustrating some components of an X-ray detector according to an embodiment of the disclosure;

FIG. 8B is a plan view of the components illustrated in FIG. 8A;

FIG. 9A is a graph of an acceleration value of an X-ray detector, which is measured over time by a shock-detecting sensor to which a shock-absorbing member is not applied;

FIG. 9B is a graph of an acceleration value of an X-ray detector, which is measured over time by a shock-detecting sensor to which the shock-absorbing member is applied, according to an embodiment of the disclosure;

FIG. 10A illustrates an acceleration value measured by a shock-detecting sensor, to which a shock-absorbing member is not applied, with respect to the number of falls of an X-ray detector;

FIG. 10B illustrates an acceleration value measured by a shock-detecting sensor, to which the shock-absorbing member is applied, with respect to the number of falls of an X-ray detector, according to an embodiment of the disclosure;

FIG. 11 is a block diagram of a configuration of an X-ray system according to an embodiment of the disclosure;

FIG. 12 is a graph of an acceleration value of an X-ray detector measured by a shock-detecting sensor with respect to time, according to an embodiment of the disclosure; and

FIG. 13 is a flowchart of a method of operating an X-ray system according to an embodiment of the disclosure.

## Best Mode for Carrying out the Invention

According to an embodiment of the disclosure, an X-ray detector includes features defined in the appended independent system and method claims.

## Mode for the Invention

The principle of the disclosure is explained and embodiments are disclosed so that the scope of the disclosure is clarified and one of ordinary skill in the art to which the disclosure pertains implements the disclosure. The disclosed embodiments may have various forms.

Like reference numerals refer to like elements throughout the specification. This specification does not describe all the elements of the embodiments, and duplicate contents of the general contents or embodiments in the technical field of the disclosure will be omitted. The terms "part" and "portion" as used herein may be embodied in software or hardware. According to embodiments of the disclosure, a plurality of "parts" or "portions" may be embodied as a single unit or a single element. Alternatively, a single 'part' or a single 'portion' may include a plurality of units or a plurality of elements. Hereinafter, the working principle and embodiments of the disclosure will be described with reference to the accompanying drawings.

In the present specification, an image may include a medical image obtained by a medical scanning apparatus, such as a magnetic resonance photographing (MRI) apparatus, a computed tomography (CT) apparatus, an ultrasound photographing apparatus, or an X-ray photographing apparatus.

In this specification, an 'object' is an object of photography and may be a person, an animal, or a part thereof. For example, the object may include a part of the body (an internal organ) or a phantom.

Throughout the disclosure, the expression "at least one of a, b or c" indicates only a, only b, only c, both a and b, both a and c, both b and c, all of a, b, and c, or variations thereof.

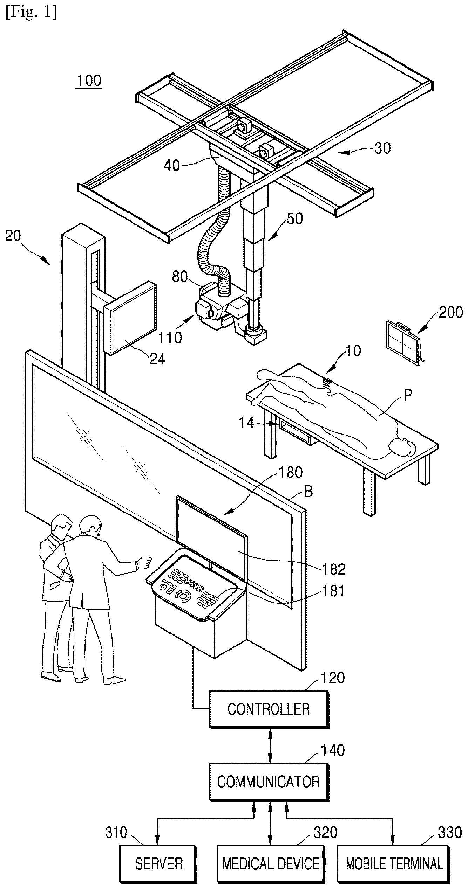

FIG. 1 is a perspective diagram showing a configuration of an X-ray apparatus 100 according to an embodiment of the disclosure. The X-ray apparatus 100 of FIG. 1 is a fixed X-ray apparatus, as an example. However, the disclosure is not limited thereto.

Referring to FIG. 1, the X-ray apparatus 100 may include an X-ray irradiator 110 for generating and irradiating an X-ray, an X-ray detector 200 for detecting an X-ray that is irradiated from the X-ray irradiator and transmitted through an object, and a workstation 180 that receives a command from a user and provides information to the user. The X-ray apparatus 100 may include a controller 120 for controlling the X-ray apparatus 100 according to an input command and a communicator 140 for communicating with an external apparatus.

Some or all of the components of the controller 120 and the communicator 140 may be included in the workstation 180 or provided separately from the workstation 180.

The X-ray irradiator 110 may include an X-ray source for generating an X-ray and a collimator for adjusting an irradiation area of the X-ray generated by the X-ray source.

A guide rail 30 may be installed on the ceiling of an examination room where the X-ray apparatus 100 is disposed. The X-ray irradiator 110 may be moved to a location corresponding to location of a target object P by connecting the X-ray irradiator 110 to a moving carriage 40 moving along the guide rail 30. The moving carriage 40 and the X-ray irradiator 110 may be connected to each other through a foldable post frame 50, so that the height of the X-ray irradiator 110 may be adjusted.

The workstation 180 may include an input unit 181 for receiving commands from a user and a display 182 for displaying information.

The input unit 181 may receive commands regarding a scanning protocol, scanning conditions, scanning timing, a position control of the X-ray irradiator 110, etc. The input unit 181 may include a keyboard, a mouse, a touch screen, a voice recognizer, etc.

The display 182 may display a screen image for guiding an input of a user, an X-ray image, a screen image showing the state of the X-ray apparatus 100, etc.

The controller 120 may control a scanning timing and scanning conditions of the X-ray irradiator 110 according to a command input from the user and may generate a medical image using image data received from the X-ray detector 200. Furthermore, the controller 120 may control the position or posture of mounts 14 and 24 to which the X-ray irradiator 110 or the X-ray detector 200 is mounted according to the scanning protocol and a position of the target object P.

The controller 120 may include a memory for storing a program for performing the above-described operation and other operations and a processor for executing the stored program. The controller 120 may include a single processor or a plurality of processors. In the latter case, a plurality of processors may be integrated on a single chip or may be physically separated from one another.

The X-ray apparatus 100 may be connected to an external device (e.g., an external server 310, a medical device 320, and a mobile terminal 330 (e.g., a smart phone, a tablet PC, a wearable device, etc.)) via the communicator 140 and transmit or receive data.

The communicator 140 may include at least one component that enables communication with an external device. For example, the communicator 140 may include at least one of a short-range communication module, a wired communication module, or a wireless communication module.

Furthermore, the communicator 140 may receive a control signal from an external device and transmit the received control signal to the controller 120, so that the controller 120 controls the X-ray apparatus 100 according to the received control signal.

Furthermore, the controller 120 may control an external device according to the control signal of the controller 120 by transmitting a control signal to the external device through the communicator 140. For example, the external device may process data of the external device according to the control signal of the controller 120 received via the communicator 140.

Furthermore, the communicator 140 may further include an internal communication module that enables communication among the components of the X-ray apparatus 100. Because a program for controlling the X-ray apparatus 100 may be installed on the external device, the program may include an instruction for performing some or all of the operations of the controller 120.

The program may be installed in the mobile terminal 330 in advance or the user of the mobile terminal 330 may download the program from the server that provides applications and install the program. The server providing applications may include a recording medium in which the program is stored.

The X-ray detector 200 may be a fixed X-ray detector fixed to a stand 20 or a table 10, may be detachably mounted to the mounts 14 and 24, or may be a portable X-ray detector that may be used at an arbitrary location. When the X-ray detector 200 is a portable X-ray detector, the X-ray detector 200 may be of a wire type or a wireless type depending on data transmission methods and power supply methods.

The X-ray detector 200 may be included as an element of the X-ray apparatus 100 or may not be included. In the latter case, the X-ray detector 200 may be registered to the X-ray apparatus 100 by a user. Furthermore, in both cases, the X-ray detector 200 may be connected to the controller 120 through the communicator 140 and receive a control signal or transmit image data.

A sub-user interface 80 for providing information to a user and receiving a command from a user may be provided at one end of the X-ray irradiator 110, where some or all of functions to be performed by the input unit 181 and the display 182 of the workstation 180 may be performed by the sub-user interface 80.

When all or some of the components of the controller 120 and the communicator 140 are provided separately from the workstation 180, they may be included in the sub-user interface 80 provided in the X-ray irradiator 110.

Although FIG. 1 shows a fixed X-ray apparatus connected to the ceiling of an examination room, the X-ray apparatus 100 may have various structures within a range that is obvious to one of ordinary skill in the art, such as a C-arm type X-ray apparatus and a mobile X-ray apparatus.

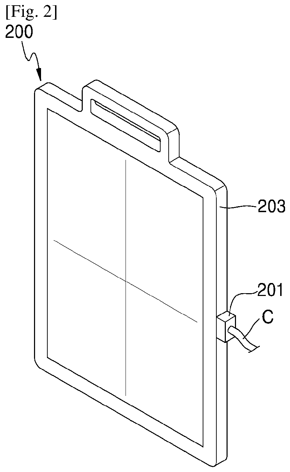

FIG. 2 is a perspective view of a portable X-ray detector according to an embodiment of the disclosure.

As described above, the X-ray detector 200 used in the X-ray apparatus 100 may be implemented as a portable X-ray detector. In this case, the X-ray detector 200 may include a battery for supplying power and operate wirelessly. Alternatively, as shown in FIG. 2, the X-ray detector 200 may operate as a charging port 201 is connected to a separate power supply unit by a cable C.

Inside a case 203 constituting the outer appearance of the X-ray detector 200, a detection element for detecting an X-ray and converting it into image data, a memory for temporarily or non-temporarily storing the image data, a communication module for receiving a control signal from the X-ray apparatus 100 or transmitting image data to the X-ray apparatus 100, and a battery may be provided. Furthermore, image correction information regarding the X-ray detector 200 and unique identification information regarding the X-ray detector 200 may be stored in the memory, and identification information stored during communication with the X-ray apparatus 100 may be transmitted together with the image correction information regarding the X-ray detector 200 and the unique identification information regarding the X-ray detector 200.

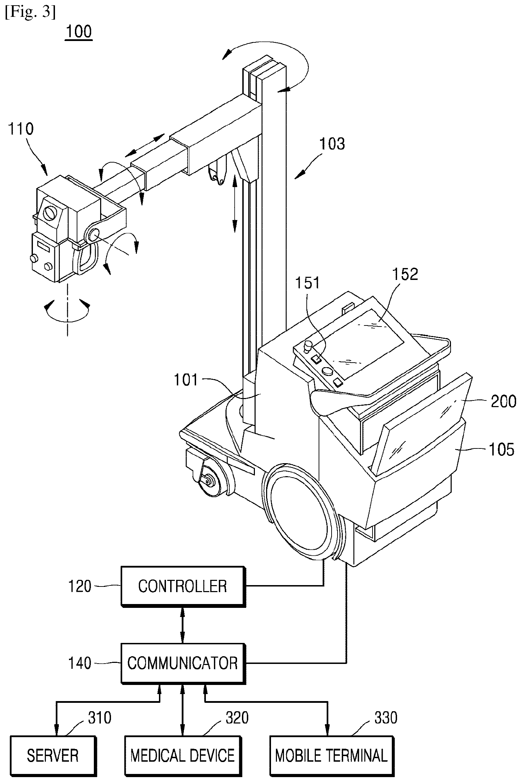

FIG. 3 is a perspective view of a mobile X-ray apparatus according to an embodiment of the disclosure.

The same reference numerals as those in FIG. 1 denote the same functions, and thus redundant description of the reference numerals in FIG. 1 will be omitted.

An X-ray apparatus may be implemented not only as the ceiling type as described above, but also as a mobile type. When the X-ray apparatus 100 is implemented as a mobile X-ray apparatus, a main body 101 to which the X-ray irradiator 110 is connected may freely move and the an arm 103 interconnecting the X-ray irradiator 110 and the main body 101 may also be rotated and linearly move, and thus the X-ray irradiator 110 may freely move in the three-dimensional (3D) space.

The main body 101 may include a storage 105 for storing the X-ray detector 200. Furthermore, a charging terminal capable of charging the X-ray detector 200 is provided in the storage 105, so that the X-ray detector 200 may be stored while being charged.

An input unit 151, a display 152, the controller 120, and the communicator 140 may be provided in the main body 101. Image data obtained by the X-ray detector 200 may be transmitted to the main body 101 and displayed on the display 152 or transmitted to an external device through the communicator 140.

The controller 120 and the communicator 140 may be provided separately from the main body 101 and only some of the components of the controller 120 and the communicator 140 may be provided in the main body 101.

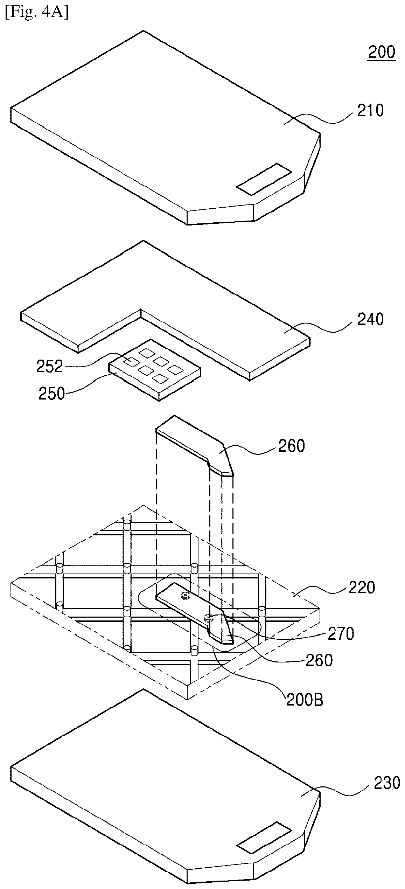

FIG. 4A is an exploded perspective view showing components of an X-ray detector 200 according to an embodiment.

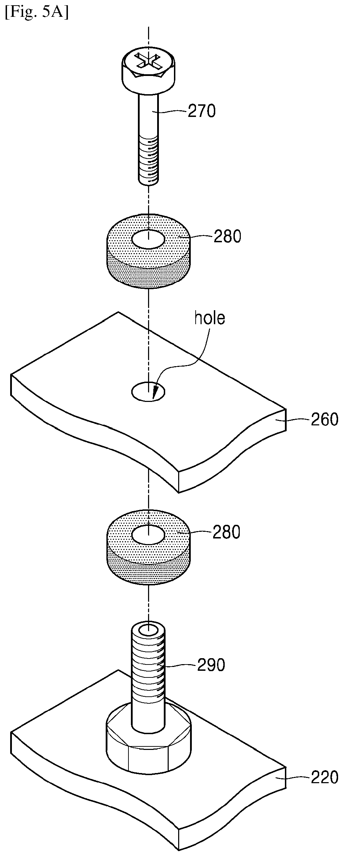

Referring to FIG. 4A, the X-ray detector 200 may include a top cover 210, a substrate support 220, a bottom cover 230, a printed circuit board (PCB) substrate 240, a detector element array 250, and a shock-detecting sensor module 260. The shock-detecting sensor module 260 may be fixedly attached to the substrate support 220 via a screw 270 and a protruding coupling member (290 of FIG. 5A). The X-ray detector 200 does not include only the components shown in FIG. 4A. According to an embodiment of the disclosure, the X-ray detector 200 may further include a scintillator layer that absorbs X-rays that have passed through an object to emit light. According to an embodiment of the disclosure, when the X-ray detector 200 is implemented as a portable X-ray detector, the X-ray detector 200 may include a battery therein.

The top cover 210 and the bottom cover 230 may encase the X-ray detector 200 by enclosing and sealing the components included therein. The top cover 210 and the bottom cover 230 serve as a casing that accommodates the components of the X-ray detector 200 therein and protects the components from being damaged when the X-ray detector 200 is dropped or is subjected to an impact. A handle may be provided on the top cover 210 and the bottom cover 230.

The substrate support 220 may be encased by the top cover 210 and the bottom cover 230, and has the PCB substrate 240, the detector element array 250, and the shock-detecting sensor module 260 mounted thereonto. Coupling parts are formed on the substrate support 220 to protrude upward in cross shapes, and a protrusion may be formed at a point where the coupling parts intersect each other. The protrusion may protrude upwards by a predetermined height to fit into a hole formed in the shock-detecting sensor module 260. However, the shape of the substrate support 220 shown in FIG. 4A is merely an example and is not limited to that shown in FIG. 4A.

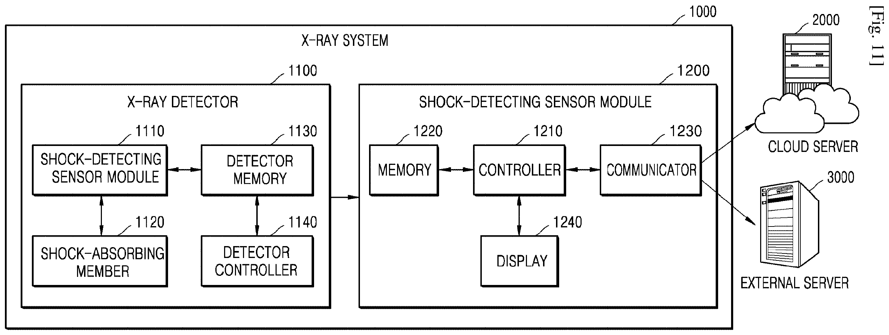

At least one processor and a memory may be mounted on the PCB substrate 240. The at least one processor performs logic operations of the X-ray detector 200 and controls the components thereof, and the memory stores data values. The at least one processor may calculate an impulse applied to the X-ray detector 200 by using an instantaneous acceleration value measured by the shock-detecting sensor module 260. Furthermore, according to an embodiment of the disclosure, the at least one processor may include a communication module that transmits the calculated impulse to an external cloud server. The memory may store an instantaneous acceleration value measured by the shock-detecting sensor module 260. According to an embodiment of the disclosure, the memory may store at least one of the number of times an instantaneous acceleration value measured by the shock-detecting sensor module 260 is greater than or equal to a preset value, information about a time when or a place where the measured instantaneous acceleration value is greater than or equal to the preset value. Information stored in the memory may be used to generate information about usage history of the X-ray detector 200, as described in more detail below with reference to FIG. 11.

The detector element array 250 may detect light emitted by the scintillator layer. The detector element array 250 may have a plurality of detector elements 252 mounted thereon. The detector elements 252 may respectively correspond to pixels in a reconstructed image. Each of the detector elements 252 may include a photosensitive region and an electronic circuit region. Each of the detector elements 252 may detect electrons that are released in proportion to light detected during emission of X-rays toward the object and store electrical charges accumulated in the photosensitive region.

The shock-detecting sensor module 260 is secured to the substrate support 220 to detect a shock applied to the X-ray detector 200. The shock-detecting sensor module 260 may include at least one of a shock sensor, an accelerometer, a geomagnetic sensor, or a gyroscopic sensor. The shock-detecting sensor module 260 may be implemented as a module including a substrate and the above-described sensors, but is not limited thereto.

According to an embodiment of the disclosure, at least one of a shock sensor, an accelerometer, a geomagnetic sensor, or a gyroscopic sensor may be directly mounted onto the PCB substrate 240. According to an embodiment of the disclosure, the shock-detecting sensor module 260 may include an accelerometer that measures an instantaneous acceleration value along each of the X- Y- and Z-axes.

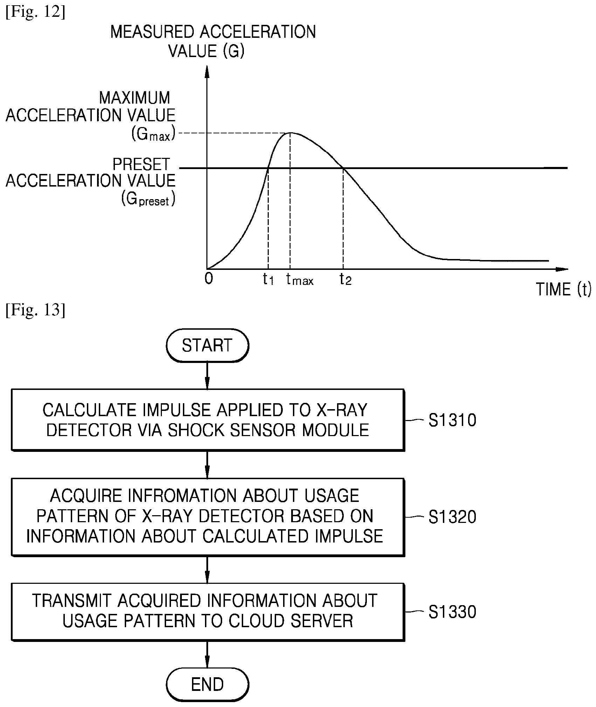

The shock-detecting sensor module 260 may not only detect a shock applied to the X-ray detector 200 but also calculate an impulse occurring due to the shock. According to an embodiment of the disclosure, the shock-detecting sensor module 260 may calculate an impulse by using instantaneous acceleration values measured at time points having a time interval corresponding to a preset sampling frequency. For example, the shock-detecting sensor module 260 may calculate an impulse applied to the X-ray detector by integrating instantaneous acceleration values measured over a preset time interval with respect to time. Furthermore, the shock-detecting sensor module 260 may calculate an impulse by selecting only a peak value from among measured instantaneous acceleration values. A method whereby the shock-detecting sensor module 260 calculates an impulse will be described in more detail below with reference to FIG. 12.

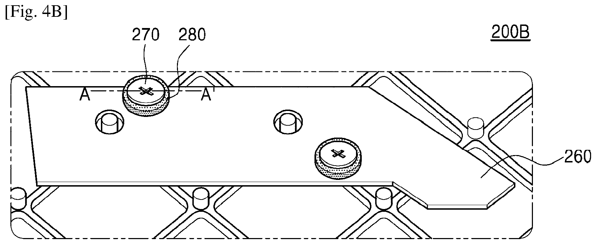

Shock-absorbing members (280 of FIG. 4B) may be provided at one side of the shock-detecting sensor module 260 to enclose portions of top and bottom surfaces of the shock-detecting sensor module 260.

FIG. 4B is an enlarged view of a part 200B of the X-ray detector 200 of FIG. 4A.

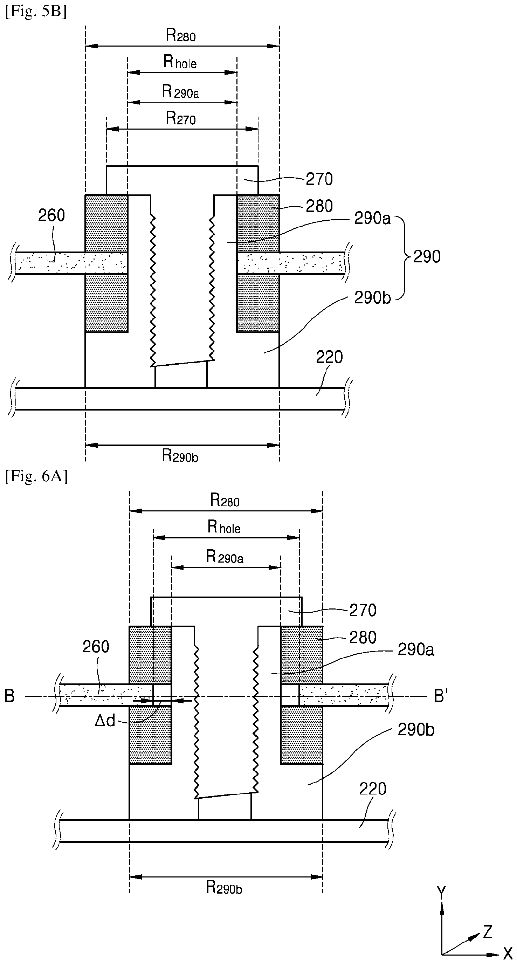

Referring to FIG. 4B, the shock-detecting sensor module 260 may be coupled to the substrate support 220 via the screw 270. According to an embodiment of the disclosure, the shock-detecting sensor module 260 may be fixedly coupled to the substrate support 220 via the screw 270 and the protruding coupling member (290 of FIGS. 5A and 5B) having a screw hole and further protruding upward by a predetermined height.

The shock-absorbing members 280 may be respectively provided between the shock-detecting sensor module 260 and the screw 270 and between the shock-detecting sensor module 260 and the protruding coupling member 290. In other words, the shock-absorbing members 280 may be provided to surround portions of the top and bottom surfaces of one side of the shock-detecting sensor module 260. The shock-absorbing members 280 may absorb a shock applied to the X-ray detector 200. According to an embodiment of the disclosure, the shock-absorbing members 280 may absorb a shock applied to the shock-detecting sensor module 260 due to a fall of the X-ray detector 200 or external shock applied thereto.

The shock-absorbing member 280 may be formed of an elastic material capable of reducing instantaneous shocks. For example, the shock-absorbing member 280 may be formed of at least one of an elastic material, a viscoelastic material, plastic, rubber, gel, carbon graphite, polycarbonate, or polyvinyl chloride (PVC). However, examples of a material constituting the shock-absorbing member 280 are not limited thereto.

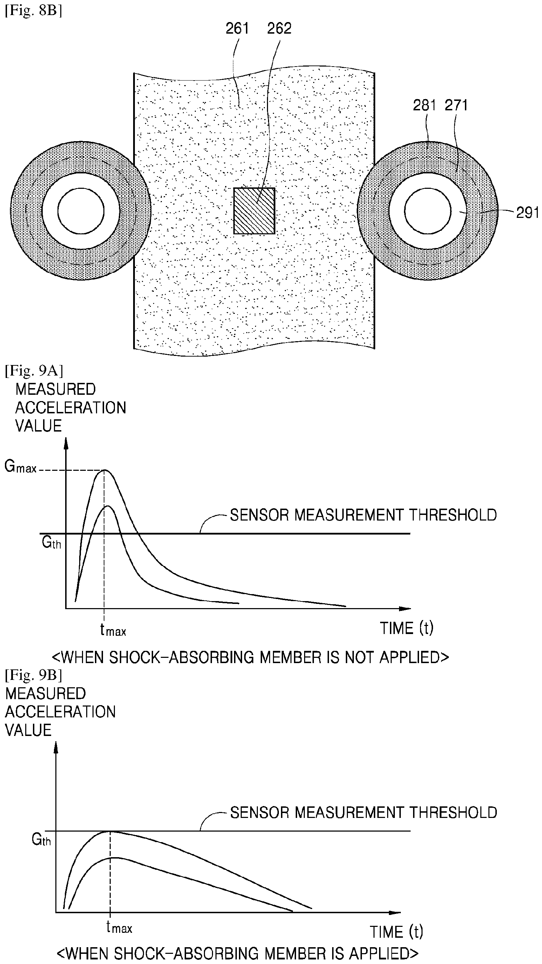

By arranging the shock-absorbing members 280 at one side of the shock-detecting sensor module 260, it is possible to reduce instantaneous momentum, i.e., an acceleration value, measurable by the shock-detecting sensor module 260. Due to the presence of the shock-absorbing member 280, a maximum instantaneous acceleration value measurable by the shock-detecting sensor module 260 may not exceed a measurement limit for the shock-detecting sensor module 260, and an allowable measurement range may be expanded. In other words, the shock-absorbing member 280 may be designed to transmit a shock applied to the X-ray detector 200 due to a fall of the X-ray detector 200 or external shock thereto to the shock-detecting sensor module 260, thereby attenuating the shock itself. Thus, an instantaneous acceleration value that is measured by the shock-detecting sensor module 260 may not be saturated. The presence of the shock-absorbing member 280 may reduce a value of shock instantaneously applied to the X-ray detector 200 while maintaining an impulse applied over a specific time period, as described in more detail below with reference to FIGS. 9A and 9B.

The shock-absorbing member 280 may perform filtering with respect to a high frequency signal caused by vibrations that occur due to a shock applied to the X-ray detector 200. For example, when the X-ray detector 200 is dropped, a signal generated due to the fall of the X-ray detector 200 may include a primary signal composed of low frequencies and induced by an impact itself and a secondary signal induced by vibrations composed of high frequencies. In this case, the primary signal is a signal of the impact itself, and the secondary signal acts as noise. The shock-absorbing member 280 may perform a function of a mechanical filter by attenuating a noise signal composed of high frequencies.

FIG. 5A is an exploded perspective view showing some components of the X-ray detector 200 according to an embodiment of the disclosure.

Referring to FIG. 5A, a hole is formed in the shock-detecting sensor module 260, and the protruding coupling member 290 passes through the hole and is then coupled with the screw 270 such that the shock-detecting sensor module 260 may be fixedly secured onto the substrate support 220. In other words, the hole in the shock-detecting sensor module 260 is formed to surround a protruding part of the protruding coupling member 290. The protruding coupling member 290 has therein a screw hole to engage with the screw 270.

The shock-absorbing members 280 may be respectively attached onto the top and bottom surfaces of the shock-detecting sensor module 260. The hole formed in the shock-detecting sensor module 260 may have a diameter greater than or equal to that of the protruding part of the protruding coupling member 290.

For example, the shock-absorbing member 280 may be formed of at least one of an elastic material, a viscoelastic material, plastic, rubber, gel, carbon graphite, polycarbonate, or PVC, but is not limited thereto.

FIG. 5B is a cross-sectional view taken along line A-A' of FIG. 4B.

Referring to FIG. 5B, the protruding coupling member 290 may include a protruding part 290a having a screw hole and protruding by a predetermined height and a coupling part 290b that is secured to the substrate support 220 to support the protruding part 290a. A diameter $R_{hole}$ of a hole in the shock-detecting sensor module 260 is equal to a diameter $R_{290a}$ of the protruding part 290a, and the shock-detecting sensor module 260 may contact the protruding part 290a via the hole.

The shock-absorbing members 280 may be respectively provided on top and bottom surfaces of the shock-detecting sensor module 260. A diameter $R_{280}$ of the shock-absorbing member 280 may be less than or equal to a diameter $R_{290b}$ of the coupling part 290b. The shock-absorbing member 280 positioned on the bottom surface of the shock-detecting sensor module 260 may be supported by the coupling part 290b of the protruding coupling member 290, and an uppermost surface of the shock-absorbing member 280 positioned on the top surface of the shock-detecting sensor module 260 may be fixed by a head of the screw 270.

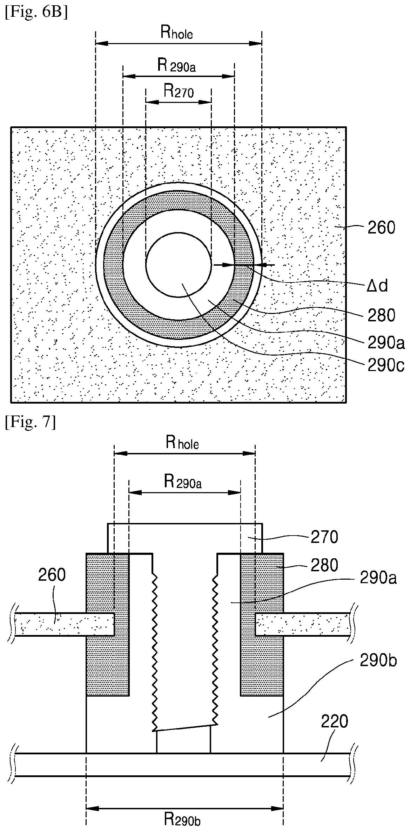

FIG. 6A is a cross-sectional view illustrating some components of the X-ray detector 200 according to an embodiment of the disclosure, and FIG. 6B is a plan view of the components illustrated in FIG. 6A.

Referring to FIG. 6A, a hole is formed in the shock-detecting sensor module 260, and the protruding part 290a of the protruding coupling member 290 penetrates through the hole to be coupled with the screw 270. Thus, the shock-detecting sensor module 260 may be attached onto the substrate support 220. The shock-absorbing members 280 may be respectively arranged on top and bottom surfaces of the shock-detecting sensor module 260.

In the embodiment shown in FIG. 6A, a diameter $R_{hole}$ of the hole in the shock-detecting sensor module 260 may be greater than a diameter $R_{290a}$ of the protruding part 290a of the protruding coupling member 290. In other words, the hole formed in the shock-detecting sensor module 260 may not contact the protruding part 290a of the protruding coupling member 290 but be spaced apart therefrom by a predetermined length $\Delta d$. A doughnut-shaped hollow space with a thickness corresponding to the predetermined length $\Delta d$ may be created between the protruding part 290a and the hole in the shock-detecting sensor module 260.

Because the shock-absorbing members 280 are provided only on the top and bottom surfaces of the shock-detecting sensor module 260, a shock applied in a Z-axis direction due to a fall of the X-ray detector 200 or external shock thereto may be attenuated. However, this arrangement may not be effective in attenuating shocks applied in X- and Y-axis directions. When the hole formed in the shock-detecting sensor module 260 is to contact the protruding part 290a as in the embodiment described with reference to FIG. 5B, a measurement value from the shock-detecting sensor module 260 may exceed a limit of shock values measurable by the shock-detecting sensor module 260 due to shocks applied in the X- and Y-axis directions, and thus the measurement value may be saturated. Unlike in the embodiment described with reference to FIG. 5B, according to the embodiment shown in FIG. 6A, the diameter $R_{hole}$ of the hole in the shock-detecting sensor module 260 may be made larger than the diameter $R_{290a}$ of the protruding part 290a of the protruding coupling member 290, and the hole may be spaced apart therefrom by a predetermined length $\Delta d$. Due to this configuration, shocks applied in the X- and Y-axis directions may not directly affect measurements of a shock value. Thus, it is possible to attenuate a value of instantaneous shock transmitted to the shock-detecting sensor module 260 and expand a range of shock values measurable by the shock-detecting sensor module 260, thereby allowing for accurate calculation of an impulse.

FIG. 6B is a plan view taken along line B-B' of FIG. 6A.

Referring to FIG. 6B, the diameter $R_{290a}$ of the protruding part 290a of the protruding coupling member 290 may be greater than a diameter R270 of the screw hole but less than the diameter $R_{hole}$ of the hole in the shock-detecting sensor module 260. The shock-absorbing member 280 with a predetermined thickness $\Delta d$ is shown as a doughnut shape positioned between an outer periphery of the protruding part 290a and the hole in the shock-detecting sensor module 260. Because the diameter $R_{hole}$ of the hole in the shock-detecting sensor module 260 is greater than the

diameter $R_{290a}$ of the protruding part 290a and the hole is spaced apart from the protruding part 290a by the predetermined length Δd instead of contacting the protruding part 290a, the shock-absorbing member 280 may be exposed as a doughnut shape in the plan view. As described above, because the hole in the shock-detecting sensor module 260 does not directly contact the protruding part 290a but is spaced apart therefrom, it is possible to attenuate an instantaneous shock value due to shocks applied in the X- and Y-axis directions.

FIG. 7 is a cross-sectional view illustrating some components of the X-ray detector 200 according to an embodiment of the disclosure.

Referring to FIG. 7, the shock-detecting sensor module 260 may have a hole therein as shown in FIG. 6A, and the protruding part 290a of the protruding coupling member 290 passes through the hole in the shock-detecting sensor module 260 and is then coupled thereto by the screw 270 such that the shock-detecting sensor module 260 may be secured onto the substrate support 220. The shock-absorbing members 280 may be respectively provided on top and bottom surfaces of the shock-detecting sensor modules 260.

In the embodiment shown in FIG. 7, a diameter $R_{hole}$ of the hole in the shock-detecting sensor module 260 may be greater than a diameter $R_{290a}$ of the protruding part 290a of the protruding coupling member 290. In other words, the hole formed in the shock-detecting sensor module 260 may not contact the protruding part 290a of the protruding coupling member 290 but be spaced apart therefrom by a predetermined length Δd. However, the embodiment shown in FIG. 7 is different from the embodiment described with reference to FIG. 6A in that a space between the hole and the protruding part 290a is filled with the shock-absorbing members 280. In other words, the shock-absorbing members 280 may be provided to fill the space between the shock-detecting sensor module 260 and the protruding part 290a.

According to the embodiment shown in FIG. 7, the hole formed in the shock-detecting sensor module 260 does not contact the protruding part 290a, and the shock-absorbing members 280 are formed in the space between the hole and the protruding part 290a. Thus, it is possible to attenuate an instantaneous shock applied in the X- and Y-axis directions due to a fall of the X-ray detector 200 or external shock applied thereto. Due to the attenuation of instantaneous shock, a maximum instantaneous shock value measurable by the shock-detecting sensor module 260 may be smaller than a measurement limit, and thus, a range of shock values measurable by the shock-detecting sensor module 260 may be expanded.

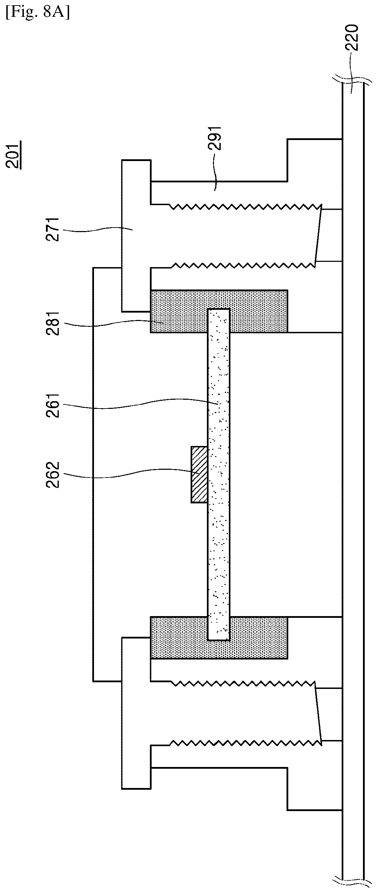

FIG. 8A is a cross-sectional view illustrating some components of an X-ray detector 201 according to an embodiment of the disclosure, and FIG. 8B is a plan view of the components illustrated in FIG. 8A.

Referring to FIGS. 8A and 8B, the X-ray detector 201 may include a substrate support 220, a PCB substrate 261, a shock-detecting sensor 262, and a shock-absorbing member 281. The PCB substrate 261 and the shock-absorbing member 281 may be fixedly attached onto the substrate support 220 via screws 271 and protruding coupling members 291.

The shock-detecting sensor 262 may be mounted on the PCB substrate 261 and detect shock applied to the X-ray detector 201 and calculate an impulse occurring due to the shock. For example, the shock-detecting sensor 262 may include at least one of an accelerometer, a geomagnetic sensor, or a gyroscopic sensor, but is not limited thereto. According to an embodiment of the disclosure, at least one processor for performing logic operations of the X-ray detector 201 and controlling the components thereof and a memory for storing data values may also be mounted on the PCB substrate 261.

The shock-absorbing members 281 may be positioned at both ends of the PCB substrate 261. According to an embodiment of the disclosure, the shock-absorbing member 281 may be provided at either end of the PCB substrate 261 to surround a top surface, a bottom surface, and side surfaces of the either end thereof. According to an embodiment of the disclosure, the shock-absorbing member 281 may absorb a shock applied to the PCB substrate 261 with the shock-detecting sensor 262 mounted thereon due to a fall of the X-ray detector 201 or external shock thereto.

The shock-absorbing member 281 may be formed of an elastic material capable of reducing instantaneous shocks. For example, the shock-absorbing member 281 may be formed of at least one of an elastic material, a viscoelastic material, plastic, rubber, gel, carbon graphite, polycarbonate, or PVC. However, examples of a material constituting the shock-absorbing member 281 are not limited thereto.

Referring to FIG. 8B, the screw 271 of a circular shape and the protruding coupling member 291 are coupled at either end of the PCB substrate 261, and the shock-absorbing member 281 is concentric with the screw 271 and the protruding coupling member 291. However, embodiments of the disclosure are not limited thereto. According to an embodiment of the disclosure, the PCB substrate 261 may be secured to the substrate support 220 by fixing a quadrangular (not circular) coupling member into a groove.

In the embodiments shown in FIGS. 8A and 8B, the shock-detecting sensor 262 is not implemented as a module but is mounted directly on the PCB substrate 261, and the shock-absorbing member 281 is positioned at either end of the PCB substrate 261 to surround all sides, i.e., top, bottom, and side surfaces of the either end. Due to this configuration, it is possible to attenuate a value of instantaneous shock transmitted to the shock-detecting sensor 262 when the X-ray detector 200 is dropped or is subjected to an external shock. Thus, a maximum instantaneous shock value measurable by the shock-detecting sensor 262 may be smaller than a measurement limit, and a measurement value from the shock-detecting sensor 262 may not be saturated. Accordingly, a range of shock values measurable by the shock-detecting sensor 262 may be expanded.

FIG. 9A is a graph of an acceleration value of an X-ray detector, which is measured over time by a shock-detecting sensor to which a shock-absorbing member is not applied, and FIG. 9B is a graph of an acceleration value of an X-ray detector, which is measured over time by a shock-detecting sensor whose top and bottom surfaces are surrounded by a shock-absorbing member, according to an embodiment of the disclosure.

The graphs of FIGS. 9A and 9B each show an acceleration value measured by a shock-detecting sensor, and an impulse applied to the X-ray detector may be calculated using an acceleration value measured by the shock-detecting sensor. In detail, an impulse I due to a fall of the X-ray detector or shock applied thereto may be calculated by multiplying a momentum P by the elapsed time $\Delta t$ as defined by Equation 1 below:

[Math. 1]

$$\vec{I} = \vec{p} \times \Delta t$$

Because the momentum P is the product of mass and velocity, and the velocity is calculated as the product of acceleration a and time t, the impulse I may be proportional to acceleration a. The shock-detecting sensor may include at least one of an accelerometer, a geomagnetic sensor, or a gyroscopic sensor, and measure instantaneous acceleration values along three axes, i.e., the X- Y- and Z-axes, at respective time points having a time interval corresponding to a sampling frequency by using the accelerometer.

According to an embodiment of the disclosure, the shock-detecting sensor may calculate an acceleration a by using a root mean square (RMS) of values measured by a three-axis accelerometer measuring accelerations along the X-, Y-, and Z-axes, as defined by Equation 2 below:

[Math. 2]

$$a = \sqrt{X^2 + Y^2 + Z^2}$$

According to another embodiment of the disclosure, the shock-detecting sensor may calculate an acceleration a by using a vector sum of values measured by a three-axis accelerometer measuring accelerations along the X- Y-, and Z-axes, as defined by Equation 3 below:

[Math. 3]

$$a = \sum(|X| + |Y| + |Z|)$$

Referring to FIG. 9A, when an X-ray detector is dropped or is subjected to external shock, a maximum acceleration value measured by the shock-detecting sensor, to which a shock-absorbing member is not applied, may exceed a measurement limit for the shock-detecting sensor. In general, because a shock-detecting sensor mounted in an X-ray detector has a compact size and a low measurement limit, when an acceleration value momentarily increases due to a fall of the X-ray detector or shock applied thereto, a measured acceleration value may reach a measurement threshold $G_{th}$ and then become saturated. When a measured acceleration value reaches the measurement threshold $G_{th}$ measurable by the shock-detecting sensor, a maximum acceleration value at a time when shock is applied may be measured as the measurement threshold $G_{th}$, and thus, distortion of a measurement value may occur.

For example, a maximum acceleration value that needs to be measured at a particular time $t_{max}$ after the X-ray detector is dropped may be measured as the measurement threshold $G_{th}$ at the time $t_{max}$ due to a measurement limit for the shock-detecting sensor.

Referring to FIG. 9B, shock transmitted to the shock-detecting sensor due to a fall of the X-ray detector or external shock applied thereto may be attenuated by the shock-absorbing member. Thus, an acceleration value measured by the shock-detecting sensor may be reduced.

For example, an acceleration value at a particular time $t_{max}$ when a maximum acceleration value is to be measured may be equal to or less than a sensor measurement threshold $G_{th}$. Because the shock-absorbing member is attached to the shock-detecting sensor, saturation may not occur in the shock-detecting sensor, thereby preventing distortion at which the maximum acceleration value equals the sensor measurement threshold $G_{th}$.

As seen in the graphs of FIGS. 9A and 9B, instantaneous acceleration values measured at respective time points by the shock-detecting sensors to which the shock-absorbing member is and not applied are different, whereas impulses calculated by the shock-detecting detectors as the integral of an instantaneous acceleration value over time have approximately the same values. Thus, even when a shock-absorbing member is applied to a shock-detecting sensor module, remarkable distortion does not occur in a calculated impulse value.

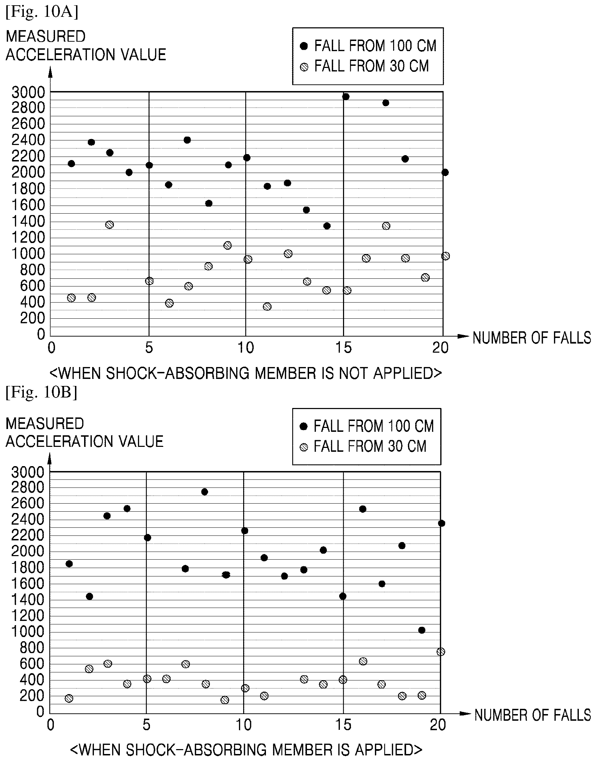

FIG. 10A illustrates an acceleration value measured by a shock-detecting sensor, to which a shock-absorbing member is not applied, with respect to the number of falls of an X-ray detector, and FIG. 10B illustrates an acceleration value measured by a shock-detecting sensor, to which a shock-absorbing member is applied, with respect to the number of falls of an X-ray detector according to an embodiment of the disclosure.

Referring to FIG. 10A, when the shock-absorbing member is not attached to the shock-detecting sensor, an acceleration value measured when the X-ray detector is dropped from a height of 30 cm is not clearly distinguishable from an acceleration value measured when the X-ray detector is dropped from a height of 100 cm. For example, when a measured acceleration value is in a range of between 1400 and 1600, acceleration values in the case of falling from the height of 30 cm and acceleration values in the case of falling from the height of 100 cm are distributed together over the range. Furthermore, when the X-ray detector is dropped from the height of 30 cm, it can be seen that a measured acceleration value in the graph of FIG. 10A is greater than a measured acceleration value in the graph of FIG. 10B.

Referring to FIG. 10B, when the shock-absorbing member is attached to the shock-detecting sensor, an acceleration value measured when the X-ray detector is dropped from a height of 30 cm is highly distinguishable from an acceleration value measured when the X-ray detector is drooped from a height of 100 cm, as compared to the case illustrated in the graph of FIG. 10A. For example, acceleration values measured when the X-ray detector is dropped from the height of 30 cm may often be observed in a range above 800 on the graph of FIG. 10A but not be observed in that range on the graph of FIG. 10B, as apparent from the Table 1 below:

[Table 1]

| | Without shock-absorbing member Fall from 30 cm | With shock-absorbing member Fall from 30 cm | Without shock-absorbing member Fall at 100 cm | With shock-absorbing member Fall at 100 cm |
|---|---|---|---|---|
| Average | 805.9 | 436.2 | 2274.4 | 2021.6 |
| Standard deviation | 295.9 | 184.1 | 640.8 | 466.0 |

Referring to the Table 1, even when the X-ray detector is dropped from the same height of 30 cm, an average acceleration value measured by the shock-detecting sensor with the shock-absorbing member attached thereto is less than an average acceleration value measured by the shock-detecting sensor without the shock-absorbing member. Furthermore, a standard deviation of acceleration values measured by the shock-detecting sensor with the shock-absorbing member attached thereto (See FIG. 10B) is smaller than a standard deviation of acceleration values measured by the shock-detecting sensor without the shock-absorbing member (See FIG. 10A).

As seen on the Table 1, when the X-ray detector is dropped from the height of 30 cm, an average acceleration value obtained when the shock-absorbing member is applied is about 370 less than an average acceleration value obtained when the shock-absorbing member is not applied. On the other hand, when the X-ray detector is dropped from the height of 100 cm, a difference between average acceleration values obtained when the shock-absorbing is and not applied may be approximately 250. This may mean that when the X-ray detector is dropped from the height of 100 cm, an acceleration value exceeding a limit measurable by the shock-detecting sensor has been measured and a measurement value itself has been saturated.

FIG. 11 is a block diagram of a configuration of an X-ray system 1000 according to an embodiment of the disclosure.

Referring to FIG. 11, the X-ray system 1000 may include an X-ray detector 1100 and a workstation 1200. According to an embodiment of the disclosure, the X-ray system 1000 may further include an X-ray irradiator emitting X-rays toward an object.

The X-ray detector 1100 may detect X-rays that are emitted by the X-ray irradiator and pass through the object. The X-ray detector 1100 may include a shock-detecting sensor module 1110, a shock-absorbing member 1120, a detector memory 1130, and a detector controller 1140.

The shock-detecting sensor module 1110 may detect a shock applied to the X-ray detector 1100 and measure an instantaneous acceleration value of the X-ray detector 1100. According to an embodiment of the disclosure, the shock-detecting sensor module 1110 may measure acceleration values at respective time points having a time interval corresponding to a preset sampling frequency. According to an embodiment of the disclosure, the shock-detecting sensor module 1110 may include a three-axis accelerometer that measures accelerations in the X- Y- and Z-axis directions. However, embodiments of the disclosure are not limited thereto, and the shock-detecting sensor module 1110 may further include at least one of a shock sensor, a geomagnetic sensor, or a gyroscopic sensor.

The shock-absorbing member 1120 may be provided at one side of the shock-detecting sensor module 1110 to surround portions of top and bottom surfaces of the shock-detecting sensor module 260. The shock-absorbing members 1120 may be respectively provided between the shock-detecting sensor module 1110 and a screw and between the shock-detecting sensor module 1110 and a protruding coupling member. An embodiment of the disclosure in which the shock-absorbing member 1120 is attached to the shock-detecting sensor module 1110 is substantially the same as any one of the embodiments of the disclosure described with reference to FIGS. 4A through 8B showing attaching of the shock-absorbing members 280 and 281, and thus, a detailed description thereof will be omitted here.

The shock-absorbing member 1120 may absorb the shock applied to the X-ray detector 1100. According to an embodiment of the disclosure, the shock-absorbing member 1120 may absorb a shock applied to the shock-detecting sensor module 1110 due to a fall of the X-ray detector 1100 or external shock thereto. The shock-absorbing member 1120 may attenuate a value of instantaneous shock transmitted to the shock-detecting sensor module 1110 to thereby expand a range of shock values measurable by the shock-detecting sensor module 1110.

The detector memory 1130 may store an instantaneous acceleration value measured by the shock-detecting sensor module 1110. The detector memory 1130 may consist of at least one of a volatile memory (e.g., dynamic random access memory (DRAM), static RAM (SRAM), synchronous DRAM (SDRAM), etc.), a non-volatile memory (e.g., one time programmable read-only memory (OTPROM), programmable ROM (PROM), erasable and programmable ROM (EPROM), electrically erasable and programmable ROM (EEPROM), mask ROM, flash ROM, etc.), a hard disk drive (HDD), or a solid state drive (SSD).

The detector controller 1140 may calculate an impulse applied to the X-ray detector 1100 by using an instantaneous acceleration value measured by the shock-detecting sensor module 1110. The detector controller 1140 may be composed of at least one processor having computational capabilities of calculating an impulse by using instantaneous acceleration values measured at respective time points. For example, the detector controller 1140 may be implemented as at least one hardware component from among a central processing unit (CPU), a microprocessor, a graphic processing unit, and an application server (AP).

According to an embodiment of the disclosure, the detector controller 1140 may calculate an impulse by integrating with respect to time acceleration values measured at respective time points having a time interval corresponding to a preset sampling frequency. According to another embodiment of the disclosure, the detector controller 1140 may calculate an impulse based on a peak value among acceleration values measured at time points corresponding to a preset sampling frequency. Furthermore, the detector controller 1140 may calculate an impulse by summing up measurement values obtained over a time interval when a measured acceleration value is greater than or equal to a preset acceleration value. According to an embodiment of the disclosure, the impulse may be calculated by a processor included in the shock-detecting sensor module 1110.

A method whereby the detector controller 1140 calculates an impulse will be described in more detail below with reference to FIG. 12.

The workstation 1200 may receive a command from a user and provide information to the user. According to an embodiment of the disclosure, the workstation 1200 may receive a calculated impulse from the X-ray detector 1100 and output information about the impulse to a display 1240. The workstation 1200 may include a controller 1210, a memory 1220, a communicator 1230, and the display 1240.

The controller 1210 may receive shock detection information from the X-ray detector 1100. For example, the shock detection information may include information about at least one of the number of occurrences of a fall of or external shock to the X-ray detector 1100, a time when the fall or external shock occurs, or a place where the fall or external shock occurs. According to an embodiment of the disclosure, the controller 1210 may acquire information about an impulse calculated by the detector controller 1140 from the X-ray detector 1100.

According to an embodiment of the disclosure, the controller 1210 may generate information about usage history of the X-ray detector 1100 based on the shock detection information received from the X-ray detector 1100. The information about usage history of the X-ray detector 1100 may be usage information regarding how many times the X-ray detector 1100 is dropped, how many times a shock greater than or equal to a threshold is applied, a time when the shock or fall occurs, a place where the shock or fall occurs, etc.

The controller 1210 may include a memory storing programs for performing the above-described operations and operations that will be described later and a processor executing the stored programs. For example, the controller 1210 may be formed as a hardware module including at least one of a CPU, a microprocessor, a graphic processing unit, RAM, or ROM. According to an embodiment of the disclosure, the controller 1210 may be implemented as a hardware component such as a field-programmable gate array (FPGA) or an application-specific integrated circuit (ASIC).

The controller 1210 may control the memory 1220 to store shock detection information received from the X-ray detector 1100 and information about usage history of the X-ray detector 1100 generated based on the shock detection information. According to an embodiment of the disclosure, the memory 1220 may also store an impulse value calculated by the detector controller 1140.

The communicator 1230 may be controlled by the controller 1210 to transmit information about usage history of the X-ray detector 1100 to a cloud server 2000 or an external server 3000. The communicator 1230 may include one or more components that enable communication with an external device including the cloud server 2000 and the external server 3000. For example, the communicator 1230 may include at least one of a short-range communication module, a wired communication module, or a wireless communication module.

The display 1240 may display a user interface (UI) for providing a user with information indicating shock-detecting due to a fall of the X-ray detector 1100 or external shock applied thereto. For example, the display 1240 may be constituted by a physical device including at least one of a liquid crystal display (LCD), a plasma display panel (PDP), an organic light-emitting display (OLED), a field emission display (FED), a light-emitting diode (LED) display, a vacuum fluorescent display (VFD), a digital light processing (DLP) display, a flat panel display (FPD), a 3D display, or a transparent display, but is not limited thereto. According to an embodiment of the disclosure, the display 1240 may be formed as a touch screen including a touch interface.

According to an embodiment of the disclosure, the workstation 1200 may further include a sound outputter configured to output an alarm sound that notifies about occurrence of a shock due to a fall of the X-ray detector 1100 or external shock applied thereto.

FIG. 12 is a graph of an acceleration value of an X-ray detector measured by a shock-detecting sensor with respect to time, according to an embodiment of the disclosure.

Referring to FIG. 12, an acceleration value G measured by a shock-detecting sensor module according to an embodiment of the disclosure starts to rapidly increase at zero (0) second and has a maximum value Gmax at a time $t_{max}$. The acceleration value G then progressively decreases to a value close to 0.

The detector controller (1140 of FIG. 11) may calculate an impulse by integrating acceleration values G measured at respective time points having a time interval corresponding to a preset sampling frequency with respect to time from the initial time to the final time. As described with reference to FIGS. 9A and 9B, an impulse is an integral of momentum over time, the momentum is the product of mass and velocity of the X-ray detector, and the velocity is the product of acceleration and time. Thus, the impulse may be calculated by integrating acceleration values G with respect to time.

According to an embodiment of the disclosure, the detector controller 1140 may calculate an impulse based on the maximum value Gmax among acceleration values measured at time points corresponding to a sampling frequency.

According to another embodiment of the disclosure, the detector controller 1140 may calculate an impulse by summing up acceleration values G measured over a time interval (an interval between time points t1 and t2) when a measured acceleration value G is greater than or equal to a preset acceleration value Gpreset. The preset acceleration value Gpreset may be set or modified based on a user input.

According to an embodiment of the disclosure, the impulse may also be calculated by a processor included in the shock-detecting sensor module (1110 of FIG. 11).

FIG. 13 is a flowchart of a method of operating an X-ray system according to an embodiment of the disclosure.

The X-ray system calculates an impulse applied to an X-ray detector via a shock-detecting sensor module (S1310). According to an embodiment of the disclosure, the shock-detecting sensor module mounted in the X-ray detector may include an accelerometer that measures accelerations along three axes, i.e., X-, Y-, and Z-axes and measure an acceleration value by using the accelerometer. The X-ray detector may calculate an impulse by integrating measured acceleration values over time, by using a maximum value among the measured acceleration values, or by summing up acceleration values measured over a time interval when an acceleration value is greater than or equal to a preset acceleration value. Because a method whereby the X-ray detector calculates an impulse applied thereto is substantially the same as that described with reference to FIG. 12, a detailed description thereof will be omitted here.

The X-ray system acquires information about usage history of the X-ray detector based on information about the calculated impulse (S1320). According to an embodiment of the disclosure, the X-ray system may receive from the X-ray detector information about at least one of the number of occurrences of a fall of or external shock to the X-ray detector, a time when the fall or external shock occurs, or a place where the fall or external shock occurs. According to an embodiment of the disclosure, the X-ray system may generate the information about usage history of the X-ray detector based on shock detection information received from the X-ray detector. The information about usage history of the X-ray detector may be usage information related to how many times the user drops the X-ray detector during use, how many times a shock greater than or equal to a threshold is applied, a time when the shock or fall occurs, a place where the shock or fall occurs, etc.

The X-ray system transmits the information about usage history of the X-ray detector to a cloud server (S1330). According to an embodiment of the disclosure, the X-ray system may transmit the information about usage history of the X-ray detector to an external server that is not the cloud server. In this case, the external server may be a server of a manufacturer of the X-ray system, but is not limited thereto.

According to an embodiment of the disclosure, the X-ray system may provide a UI for providing a user with information indicating occurrence of a shock due to a fall of the X-ray detector or external shock applied thereto. Furthermore, when shock occurs due to a fall of the X-ray detector or external shock thereto, the X-ray system may output an alarm sound to notify the user about occurrence of the shock.

The embodiments may be implemented as a software program including instructions stored in a computer-readable storage medium.

A computer may refer to a device configured to retrieve an instruction stored in the computer-readable storage medium and to operate, in response to the retrieved instruction, and may include an X-ray imaging apparatus according to embodiments of the disclosure.

The computer-readable storage medium may be provided in the form of a non-transitory storage medium. In this regard, the term 'non-transitory' means that the storage medium does not include a signal and is tangible, and the term does not distinguish between data that is semi-permanently stored and data that is temporarily stored in the storage medium.

In addition, the X-ray detector, the X-ray system, and the method of operating the X-ray system according to embodiments of the disclosure may be provided in the form of a computer program product. The computer program product may be traded, as a product, between a seller and a buyer.

The computer program product may include a software program and a computer-readable storage medium having stored thereon the software program. For example, the computer program product may include a product (e.g. a downloadable application) in the form of a software program electronically distributed by a manufacturer of the X-ray imaging apparatus or through an electronic market (e.g., Google™, Play Store™, and App Store™). For such electronic distribution, at least a part of the software program may be stored on the storage medium or may be temporarily generated. In this case, the storage medium may be a storage medium of a server of the manufacturer, a server of the electronic market, or a relay server for temporarily storing the software program.

In a system consisting of a server and a terminal (e.g., the X-ray imaging apparatus), the computer program product may include a storage medium of the server or a storage medium of the terminal. Alternatively, in a case where a third device (e.g., a smartphone) that communicates with the server or the terminal is present, the computer program product may include a storage medium of the third device. Alternatively, the computer program product may include a software program that is transmitted from the server to the terminal or the third device or that is transmitted from the third device to the terminal.

In this case, one of the server, the terminal, and the third device may execute the computer program product, thereby performing the method according to embodiments of the disclosure. Alternatively, at least two of the server, the terminal, and the third device may execute the computer program product, thereby performing the method according to embodiments of the disclosure in a distributed manner.

For example, the server (e.g., a cloud server, an artificial intelligence (AI) server, or the like) may execute the computer program product stored in the server, and may control the terminal to perform the method according to embodiments of the disclosure, the terminal communicating with the server.

As another example, the third device may execute the computer program product, and may control the terminal to perform the method according to embodiments of the disclosure, the terminal communicating with the third device. In more detail, the third device may remotely control the X-ray imaging apparatus to emit X-ray to an object, and to generate an image of an inner part of the object, based on detected radiation which passes the object and is detected in an X-ray detector.

As another example, the third device may execute the computer program product, and may directly perform the method according to embodiments of the disclosure, based on at least one value input from an auxiliary device (e.g., a gantry of CT system). In more detail, the auxiliary device may emit X-ray to an object and may obtain information of radiation which passes the object and is detected in an X-ray detector. The third device may receive an input of signal information about the detected radiation from the auxiliary device, and may generate an image of an inner part of the object, based on the input radiation information.

In a case where the third device executes the computer program product, the third device may download the computer program product from the server, and may execute the downloaded computer program product. Alternatively, the third device may execute the computer program product that is pre-loaded therein, and may perform the method according to the embodiments.

The above-described embodiments of the disclosure may be embodied in form of a computer-readable recording medium for storing computer executable instructions and data. The instructions may be stored in form of program codes and, when executed by a processor, may perform a certain operation by generating a certain program module. Also, when executed by a processor, the instructions may perform certain operations of the disclosed embodiments.

While embodiments of the disclosure have been particularly shown and described with reference to the accompanying drawings, it will be understood by those of ordinary skill in the art that various changes in form and details may be made therein without departing from the scope of the appended claims. The disclosed embodiments should be considered in descriptive sense only and not for purposes of limitation.

**Claims**

1. An X-ray system (100, 1000) comprising:

   an X-ray detector (200, 1100); and
   a workstation (180, 1200), wherein the X-ray detector (200, 1100) comprises:

   a shock-detecting sensor module (260, 1110) positioned within the X-ray detector having a first side and a second side opposite the first side, and being configured to measure an instantaneous acceleration value of the X-ray detector (200, 1100),
   a detector memory (1130) storing the instantaneous acceleration value measured by the shock-detecting sensor module (260, 1110), and
   a detector controller (1140) configured to calculate an impulse applied to the X-ray detector (200, 1100) by using the measured instantaneous acceleration value and transmit the calculated impulse to the workstation (180, 1200),
   wherein the workstation (180, 1200) is configured to receive the calculated impulse from the X-ray detector and output information about the impulse,
   **CHARACTERISED IN THAT** the X-ray detector (200, 1100) comprises:

   a shock-absorbing member (280, 1120) attached onto the first side of the shock-detecting sensor module (260, 1110) and the second side of the shock-detecting sensor module (260, 1110) positioned within the X-ray detector (200, 1100),
   wherein, in response to an instantaneous acceleration being applied to the X-ray detector (200, 1100), the shock-absorbing member (280, 1120) is configured to transmit a reduced acceleration value to the shock-detecting sensor module (260, 1110) to sense an expanded range of acceleration values experienced by the X-ray detector (200, 1100) within the measurement range of the shock-detecting sensor module (260, 1110).

2. The X-ray system (100, 1000) of claim 1, wherein the detector controller (1140) is further configured to calculate the impulse by integrating with respect to time acceleration values measured by the shock-detecting sensor module (260, 1110) at respective time points with a time interval corresponding to a preset sampling frequency.

3. The X-ray system (100, 1000) of claim 1, wherein the detector controller (1140) is further configured to calculate the impulse based on a peak value among acceleration values measured by the shock-detecting sensor module (260, 1110) at respective time points with a time interval corresponding to a preset sampling frequency.

4. The X-ray system (100, 1000) of claim 1, wherein the detector controller is further configured to calculate the impulse by summing up, from among acceleration values measured by the shock-detecting sensor module (260, 1110) at respective time points with a time interval corresponding to a preset sampling frequency, acceleration values measured over a time interval when an acceleration value is greater than or equal to a preset acceleration value.

5. The X-ray system (100, 1000) of claim 1, wherein

   the workstation (180, 1200) comprises a communication module configured to transmit data to an external cloud server by using a wireless or wired communication method, and
   the workstation (180, 1200) is further configured to transmit the impulse to the external cloud server by using the communication module.

6. The X-ray system (100, 1000) of claim 1, wherein the workstation comprises a controller configured to

   receive shock detection information including at least one of
   a number of occurrences of a fall of the X-ray detector (200, 1100) or external shock applied thereto,
   a time when the fall or external shock occurs, and
   a place where the fall or external shock occurs, and
   generate information about usage history of the X-ray detector (200, 1100) based on the received shock detection information.

7. The X-ray system (100, 1000) of claim 6, wherein

the workstation (180, 1200) further comprises a communication module configured to transmit data to an external server by using a wireless or wired communication method, and
the controller is further configured to control the communication module to transmit the generated information about the usage history to the external server.

8. The X-ray system (100, 1000) of claim 1, wherein the workstation (180, 1200) comprises a display displaying a user interface (VI) configured to provide a user with information about an occurrence of a shock due to a fall of the X-ray detector (200, 1100).

9. The X-ray system (100, 1000) of claim 1, wherein the workstation (180, 1200) comprises a sound outputter configured to output an alarm sound notifying about an occurrence of a shock due to a fall of the X-ray detector (200, 1100).

10. A method of operating the X-ray system (100, 1000) according to any one of claims 1-9, the method comprising:

calculating (S1310) an impulse applied to the X-ray detector (200, 1100) via the shock-detecting sensor module;
acquiring (S1320) information about usage history of the X-ray detector (200, 1100) based on information about the calculated impulse; and
transmitting (S1330) the acquired information about the usage history to a cloud server (2000, 3000).

11. The method of claim 10, wherein the acquiring of the information about the usage history comprises:

receiving shock detection information comprising at least one of
a number of occurrences of a fall of the X-ray detector (200, 1100) or external shock applied thereto,
a time when the fall or external shock occurs, and
a place where the fall or external shock occurs; and
generating the information about the usage history of the X-ray detector (200, 1100) based on the received shock detection information.

12. The method of claim 10, further comprising:
displaying a user interface, UI, configured to provide a user with information about an occurrence of a shock due to a fall of the X-ray detector (200, 1100) or outputting an alarm sound notifying about the occurrence of the shock.

13. A computer program product comprising a non-transitory computer-readable storage medium, wherein the non-transitory computer-readable storage medium comprises instructions executable by a processor to cause the X-ray system (100, 1000) according to any one of claims 1-9 to execute the following method steps:

calculating an impulse applied to the X-ray detector (200, 1100) via the shock-detecting sensor module (260, 1110);
acquiring information about usage history of the X-ray detector (200, 1100) based on information about the calculated impulse; and
transmitting the acquired information about the usage history to a cloud server (2000, 3000).

**Patentansprüche**

1. Röntgensystem (100, 1000), das Folgendes umfasst:

einen Röntgendetektor (200, 1100); und
eine Arbeitsstation (180, 1200), wobei der Röntgendetektor (200, 1100) Folgendes umfasst:

ein Stoßerkennungssensormodul (260, 1110), das innerhalb des Röntgendetektors positioniert ist und eine erste Seite und eine zweite Seite gegenüber der ersten Seite aufweist, und das dazu konfiguriert ist, einen momentanen Beschleunigungswert des Röntgendetektors (200, 1100) zu messen,
einen Detektorspeicher (1130), der den von dem Stoßerkennungssensormodul (260, 1110) gemessenen momentanen Beschleunigungswert speichert, und
eine Detektorsteuerung (1140), die dazu konfiguriert ist, einen an den Röntgendetektor (200, 1100) angelegten Impuls zu berechnen, indem sie den gemessenen momentanen Beschleunigungswert verwendet, und den berechneten Impuls an die Arbeitsstation (180, 1200) zu übertragen,

wobei die Arbeitsstation (180, 1200) dazu konfiguriert ist, den berechneten Impuls von dem Röntgendetektor zu empfangen und Informationen über den Impuls auszugeben,

**DADURCH GEKENNZEICHNET, DASS** der Röntgendetektor (200, 1100) Folgendes umfasst:

ein stoßabsorbierendes Element (280, 1120), das auf der ersten Seite des Stoßerkennungssensormoduls (260, 1110) und der zweiten Seite des Stoßerkennungssensormoduls (260, 1110) angebracht ist, welches Stoßerkennungssensormodul innerhalb des Röntgendetektors (200, 1100) positioniert ist,
wobei das stoßabsorbierende Element (280, 1120) als Reaktion darauf, dass eine momentane Beschleunigung auf den Röntgenstrahldetektor (200, 1100) ausgeübt wird, dazu konfiguriert ist, einen reduzierten Beschleunigungswert an das Stoßerkennungssensormodul (260, 1110) zu übertragen, um einen erweiterten Bereich von Beschleunigungswerten zu erfassen, die der Röntgendetektor (200, 1100) innerhalb des Messbereichs des Stoßerkennungssensormoduls (260, 1110) erfährt.

2. Röntgensystem (100, 1000) nach Anspruch 1, wobei die Detektorsteuerung (1140) ferner konfiguriert ist zum Berechnen des Impulses durch zeitliche Integration von Beschleunigungswerten, die durch das Stoßerkennungssensormodul (260, 1110) zu jeweiligen Zeitpunkten mit einem Zeitintervall entsprechend einer voreingestellten Abtastfrequenz gemessen wurden.

3. Röntgensystem (100, 1000) nach Anspruch 1, wobei die Detektorsteuerung (1140) ferner konfiguriert ist zum Berechnen des Impulses basierend auf einem Spitzenwert unter Beschleunigungswerten, die durch das Stoßerkennnungssensormodul (260, 1110) zu jeweiligen Zeitpunkten mit einem Zeitintervall entsprechend einer voreingestellten Abtastfrequenz gemessen wurden.

4. Röntgensystem (100, 1000) nach Anspruch 1, wobei die Detektorsteuerung ferner konfiguriert ist zum Berechnen des Impulses durch Aufsummieren von über ein Zeitintervall gemessenen Beschleunigungswerten aus Beschleunigungswerten, die von dem Stoßerkennungssensormodul (260, 1110) zu jeweiligen Zeitpunkten mit einem Zeitintervall entsprechend einer voreingestellten Abtastfrequenz gemessen werden, in dem ein Beschleunigungswert größer oder gleich einem voreingestellten Beschleunigungswert ist.

5. Röntgensystem (100, 1000) nach Anspruch 1, wobei

die Arbeitsstation (180, 1200) ein Kommunikationsmodul umfasst, das dazu konfiguriert ist, Daten unter Verwendung eines drahtlosen oder drahtgebundenen Kommunikationsverfahrens an einen externen Cloud-Server zu übertragen, und
die Arbeitsstation (180, 1200) ferner dazu konfiguriert ist, den Impuls unter Verwendung des Kommunikationsmoduls an den externen Cloud-Server zu übertragen.

6. Röntgensystem (100, 1000) nach Anspruch 1, wobei die Arbeitsstation eine Steuerung umfasst, die dazu konfiguriert ist,

Stoßerkennungsinformationen zu empfangen, einschließlich mindestens eines der Folgenden:

einer Anzahl des Auftretens eines Sturzes des Röntgendetektors (200, 1100) oder eines darauf ausgeübten externen Stoßes,
einem Zeitpunkt, zu dem der Sturz oder externen Stoß auftritt, und
einem Ort, an dem der Sturz oder externen Stoß auftritt, und

Informationen über den Nutzungsverlauf des Röntgendetektors (200, 1100) basierend auf den empfangenen Stoßerkennungsinformationen zu erzeugen.

7. Röntgensystem (100, 1000) nach Anspruch 6, wobei

die Arbeitsstation (180, 1200) ferner ein Kommunikationsmodul umfasst, das dazu konfiguriert ist, Daten unter Verwendung eines drahtlosen oder drahtgebundenen Kommunikationsverfahrens an einen externen Server zu übertragen, und
die Steuerung ferner dazu konfiguriert ist, das Kommunikationsmodul zu steuern, um die erzeugten Informationen über den Nutzungsverlauf an den externen Server zu übertragen.

8. Röntgensystem (100, 1000) nach Anspruch 1, wobei die Arbeitsstation (180, 1200) eine Anzeige umfasst, die eine Benutzerschnittstelle (UI, User Interface) anzeigt, welche dazu konfiguriert ist, einem Benutzer Informationen über das Auftreten eines Stoßes aufgrund eines Sturzes des Röntgendetektors (200, 1100) bereitzustellen.

9. Röntgensystem (100, 1000) nach Anspruch 1, wobei die Arbeitsstation (180, 1200) eine Tonausgabeeinrichtung umfasst, die konfiguriert ist, um einen Alarmton auszugeben, der über das Auftreten eines Stoßes aufgrund eines Sturzes des Röntgendetektors (200, 1100) informiert.

10. Verfahren zum Betreiben des Röntgensystems (100, 1000) nach einem der Ansprüche 1 bis 9, wobei das Verfahren Folgendes umfasst:

Berechnen (S1310) eines Impulses, der über das Stoßerkennungssensormodul an den Röntgendetektor (200, 1100) angelegt wird;
Erfassen (S1320) von Informationen über den Nutzungsverlauf des Röntgendetektors (200, 1100) basierend auf Informationen über den berechneten Impuls; und
Übertragen (S1330) der erfassten Informationen über den Nutzungsverlauf an einen Cloud-Server (2000, 3000).

11. Verfahren nach Anspruch 10, wobei das Erfassen der Informationen über den Nutzungsverlauf Folgendes umfasst:

Empfangen von Stoßerkennungsinformationen, umfassend mindestens eines von

einer Anzahl des Auftretens eines Sturzes des Röntgendetektors (200, 1100) oder eines darauf ausgeübten externen Stoßes,
einem Zeitpunkt, zu dem der Sturz oder externe Stoß auftritt, und
einem Ort, an dem der Sturz oder externe Stoß auftritt; und

Erzeugen der Informationen über den Nutzungsverlauf des Röntgendetektors (200, 1100) basierend auf den empfangenen Stoßerkennungsinformationen.

12. Verfahren nach Anspruch 10, das ferner Folgendes umfasst:
Anzeigen einer Benutzerschnittstelle (UI, User Interface), die so konfiguriert ist, dass sie einem Benutzer Informationen über das Auftreten eines Stoßes aufgrund eines Sturzes des Röntgendetektors (200, 1100) bereitstellt oder Ausgeben eines Alarmtons, der über das Auftreten des Stoßes informiert.

13. Computerprogrammprodukt, das ein übergangsloses computerlesbares Speichermedium umfasst, wobei das übergangslose computerlesbare Speichermedium Anweisungen umfasst, die von einem Prozessor ausführbar sind, um zu bewirken, dass das Röntgensystem (100, 1000) nach einem der Ansprüche 1 bis 9 die folgenden Verfahrensschritte ausführt:

Berechnen eines Impulses, der über das Stoßerkennungssensormodul (260, 1110) an den Röntgendetektor (200, 1100) angelegt wird;
Erfassen von Informationen über den Nutzungsverlauf des Röntgendetektors (200, 1100) basierend auf Informationen über den berechneten Impuls; und
Übertragen der erfassten Informationen über den Nutzungsverlauf an einen Cloud-Server (2000, 3000).

**Revendications**

1. Système radiographique (100, 1000) comprenant :

un détecteur de rayons X (200, 1100), et
un poste de travail (180, 1200) ; ledit détecteur de rayons X (200, 1100) comprenant :

un module de détection de choc (260, 1110) disposé à l'intérieur du détecteur de rayons X, présentant un premier côté et un deuxième côté opposé au premier côté, et conçu pour mesurer une valeur d'accélération instantanée du détecteur de rayons X (200, 1100),
une mémoire de détecteur (1130) dans laquelle est enregistrée la valeur d'accélération instantanée mesurée par le module de détection de choc (260, 1110), et

un contrôleur de détecteur (1140) conçu pour calculer une impulsion appliquée au détecteur de rayons X (200, 1100) au moyen de la valeur d'accélération instantanée mesurée et pour transmettre l'impulsion calculée au poste de travail (180, 1200) ;

ledit poste de travail (180, 1200) étant conçu pour recevoir l'impulsion calculée en provenance du détecteur de rayons X et pour émettre des informations concernant l'impulsion, **CARACTÉRISÉ EN CE QUE** le détecteur de rayons X (200, 1100) comprend :

un élément amortisseur (280, 1120) fixé sur le premier côté du module de détection de choc (260, 1110) et le deuxième côté du module de détection de choc (260, 1110) disposé à l'intérieur du détecteur de rayons X (200, 1100) ;

en réaction à l'application d'une accélération instantanée sur le détecteur de rayons X (200, 1100), l'élément amortisseur (280, 1120) étant conçu pour transmettre une valeur d'accélération réduite auprès du module de détection de choc (260, 1110) pour permettre la détection d'une plage élargie de valeurs d'accélération subies par le détecteur de rayons X (200, 1100) au sein de la plage de mesure du module de détection de choc (260, 1110).

2. Système radiographique (100, 1000) selon la revendication 1, dans lequel le contrôleur de détecteur (1140) est conçu en outre pour calculer l'impulsion en intégrant, en fonction du temps, des valeurs d'accélération mesurées par le module de détection de choc (260, 1110) à des instants respectifs avec un intervalle de temps correspondant à une fréquence d'échantillonnage prédéfinie.

3. Système radiographique (100, 1000) selon la revendication 1, dans lequel le contrôleur de détecteur (1140) est conçu en outre pour calculer l'impulsion compte tenu d'une valeur maximale parmi les valeurs d'accélération mesurées par le module de détection de choc (260, 1110) à des instants respectifs avec un intervalle de temps correspondant à une fréquence d'échantillonnage prédéfinie.

4. Système radiographique (100, 1000) selon la revendication 1, dans lequel le contrôleur de détecteur est conçu en outre pour calculer l'impulsion en totalisant, parmi les valeurs d'accélération mesurées par le module de détection de choc (260, 1110) à des instants respectifs avec un intervalle de temps correspondant à une fréquence d'échantillonnage prédéfinie, des valeurs d'accélération mesurées sur un intervalle de temps lors duquel une valeur d'accélération est supérieure ou égale à une valeur d'accélération prédéfinie.

5. Système radiographique (100, 1000) selon la revendication 1, dans lequel :

le poste de travail (180, 1200) comprend un module de communication conçu pour transmettre des données à un serveur nuagique externe au moyen d'un procédé de communication sans fil ou filaire, et le poste de travail (180, 1200) est conçu en outre pour transmettre l'impulsion au serveur nuagique externe au moyen du module de communication.

6. Système radiographique (100, 1000) selon la revendication 1, dans lequel le poste de travail comprend un contrôleur conçu pour :

recevoir des informations de détection de choc comprenant :

le nombre de fois que survient une chute du détecteur de rayons X (200, 1100) ou un choc externe qui y est appliqué, l'heure à laquelle survient la chute ou le choc externe, et/ou l'endroit auquel survient la chute ou le choc externe, et

générer des informations concernant l'historique d'utilisation du détecteur de rayons X (200, 1100) compte tenu des informations de détection de choc reçues.

7. Système radiographique (100, 1000) selon la revendication 6, dans lequel :

le poste de travail (180, 1200) comprend en outre un module de communication conçu pour transmettre des données à un serveur externe au moyen d'un procédé de communication sans fil ou filaire, et le contrôleur est conçu en outre pour commander le module de communication de façon à transmettre les informations concernant l'historique d'utilisation générées auprès du serveur externe.

**8.** Système radiographique (100, 1000) selon la revendication 1, dans lequel le poste de travail (180, 1200) comprend un écran affichant une interface utilisateur conçue pour fournir à un utilisateur des informations concernant la survenue d'un choc résultant d'une chute du détecteur de rayons X (200, 1100).

**9.** Système radiographique (100, 1000) selon la revendication 1, dans lequel le poste de travail (180, 1200) comprend un émetteur sonore conçu pour émettre une alarme sonore avertissant de la survenue d'un choc résultant d'une chute du détecteur de rayons X (200, 1100).

**10.** Procédé d'exploitation du système radiographique (100, 1000) selon l'une quelconque des revendications 1 à 9, le procédé comprenant :

le calcul (S1310) d'une impulsion appliquée au détecteur de rayons X (200, 1100) par le biais du module de détection de choc,
l'acquisition (S1320) d'informations concernant l'historique d'utilisation du détecteur de rayons X (200, 1100) compte tenu d'informations concernant l'impulsion calculée, et
la transmission (S1330) des informations concernant l'historique d'utilisation acquises auprès d'un serveur nuagique (2000, 3000).

**11.** Procédé selon la revendication 10, dans lequel l'acquisition des informations concernant l'historique d'utilisation comprend :

la réception d'informations de détection de choc comprenant :

le nombre de fois que survient une chute du détecteur de rayons X (200, 1100) ou un choc externe qui y est appliqué,
l'heure à laquelle survient la chute ou le choc externe, et/ou
l'endroit auquel survient la chute ou le choc externe, et

la génération des informations concernant l'historique d'utilisation du détecteur de rayons X (200, 1100) compte tenu des informations de détection de choc reçues.

**12.** Procédé selon la revendication 10, comprenant en outre :

l'affichage d'une interface utilisateur conçue pour fournir à un utilisateur des informations concernant la survenue d'un choc résultant d'une chute du détecteur de rayons X (200, 1100) ou
l'émission d'une alarme sonore avertissant de la survenue du choc.

**13.** Produit-programme informatique comprenant un support d'enregistrement non transitoire lisible par ordinateur, ledit support d'enregistrement non transitoire lisible par ordinateur comprenant des instructions exécutables par un processeur pour amener le système radiographique (100, 1000) selon l'une quelconque des revendications 1 à 9 à exécuter les étapes de procédé suivantes :

le calcul d'une impulsion appliquée au détecteur de rayons X (200, 1100) par le biais du module de détection de choc (260, 1110),
l'acquisition d'informations concernant l'historique d'utilisation du détecteur de rayons X (200, 1100) compte tenu d'informations concernant l'impulsion calculée, et
la transmission des informations concernant l'historique d'utilisation acquises auprès d'un serveur nuagique (2000, 3000).

[Fig. 1]

[Fig. 2]
200

203

201

C

[Fig. 3]

100

110

103

151    152

101

200

105

120 — CONTROLLER

140 — COMMUNICATOR

310

320

330

SERVER

MEDICAL DEVICE

MOBILE TERMINAL

[Fig. 4A]

200

210

240

252

250

260

220

270

260

200B

230

[Fig. 4B]

[Fig. 5A]

[Fig. 5B]

$R_{280}$

$R_{hole}$

$R_{290a}$

$R_{270}$

260

270

280

290a

290

290b

220

$R_{290b}$

[Fig. 6A]

$R_{280}$

$R_{hole}$

$R_{290a}$

260

270

280

290a

B ——— B'

Δd

290b

220

$R_{290b}$

Y

Z

X

[Fig. 6B]

[Fig. 7]

[Fig. 8A]

[Fig. 8B]

261    262

281  271

291

[Fig. 9A]

MEASURED
ACCELERATION
VALUE

$G_{max}$

$G_{th}$

SENSOR MEASUREMENT THRESHOLD

$t_{max}$

TIME (t)

<WHEN SHOCK-ABSORBING MEMBER IS NOT APPLIED>

[Fig. 9B]

MEASURED
ACCELERATION
VALUE

SENSOR MEASUREMENT THRESHOLD

$G_{th}$

$t_{max}$

TIME (t)

<WHEN SHOCK-ABSORBING MEMBER IS APPLIED>

[Fig. 10A]

<WHEN SHOCK-ABSORBING MEMBER IS NOT APPLIED>

[Fig. 10B]

<WHEN SHOCK-ABSORBING MEMBER IS APPLIED>

[Fig. 11]

[Fig. 12]

MEASURED ACCELERATION
VALUE (G)

MAXIMUM
ACCELERATION VALUE
($G_{max}$)

PRESET
ACCELERATION VALUE
($G_{preset}$)

0    $t_1$   $t_{max}$    $t_2$        TIME (t)

[Fig. 13]

START

CALCULATE IMPULSE APPLIED TO X-RAY
DETECTOR VIA SHOCK SENSOR MODULE — S1310

ACQUIRE INFROMATION ABOUT USAGE
PATTERN OF X-RAY DETECTOR BASED ON
INFORMATION ABOUT CALCULATED IMPULSE — S1320

TRANSMIT ACQUIRED INFORMATION ABOUT
USAGE PATTERN TO CLOUD SERVER — S1330

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180156680 A1
- US 20060071172 A1
- US 8113045 B1
- US 2015359505 A1
- US 2013205868 A1